# EUROPEAN PATENT APPLICATION

(11) **EP 1 889 565 A1**
(43) Date of publication of application: **20.02.2008**
(21) Application number: 06766501.8
(22) Date of filing: 08.06.2006
(51) Int. Cl.: A61B 1/06

(54) **MEDICAL DEVICE**

(30) Priority: 09.06.2005 JP 2005169866
(71) Applicant: Olympus Corporation, Tokyo 151-0072 (JP)
(72) Inventor: ONO, Yasuhiro, Int. Property Support Department, Hanchioji-shi, Tokyo 192-8512 (JP); TAKAHASHI, Kazumasa, Int. Property Support Department, Hachioji-shi, Tokyo 192-8512 (JP)
(74) Representative: von Hellfeld, Axel
(86) International application number: PCT/JP2006/311539
(87) International publication number: WO 2006/132323

(57) **Abstract**

A medical apparatus includes a power supply section which supplies necessary power to respective sections in the medical apparatus, a maintaining section (a delay circuit 15) which maintains supply of power to a part of processing circuits (an image processing circuit 16) until processing in the processing circuit is completed, when an operation of instructing interruption of supply of power to the respective sections is performed, and a stopping section (a control circuit 18) which immediately stops supply of power to a part of circuit elements (a CCD, an LED) when an operation of instructing interruption of supply of power to the respective sections is performed.

## Description

### Technical Field

The present invention relates to a medical apparatus, and more particularly to an electronic endoscope apparatus.

### Background Art

In a medical electronic endoscope apparatus, image data or patient information from an electronic endoscopic scope (a CCD) as an imaging section is processed by a processor (having an OS mounted therein) as an image processing device, then temporarily stored in an internal memory (an SRAM), and recorded in a PC card as an external storage medium as required. For example, JP-A 2001-178685 (KOKAI) discloses an example of such a medical electronic endoscope apparatus.

However, for example, if electric power is unexpectedly cut by a user during recording data into the PC card, supply of power to each processing section is stopped, and the data may be destroyed without being saved. Thus, capacitances of capacitors connected in parallel in a power supply device or on a power supply output side are increased so that an output voltage can be maintained in a given fixed time period by using the energy stored in the capacitors. However, increasing the capacitances of the capacitors results in increasing the size or cost of the apparatus, and capacitors with larger capacitances are required in a power supply with a large output capacity in particular, thus making it difficult to reduce the cost or the size. Further, time management is also disadvantageously difficult due to unevenness in the capacitances of the capacitors themselves.

On the other hand, a power supply voltage to the CCD mounted at a distal end of the endoscope must be shutdown simultaneously with interruption of power in terms of assuring safety. Furthermore, at the time of power-off, indication of an LED on a front panel of the processor must be immediately turned off to prevent a sense of use from being impaired for a user.

### Disclosure of Invention

Considering the above-explained matters, controlling shutdown at the time of interruption of power requires the following two functions, but a conventional technology including JP-A 2001-178685 (KOKAI) has not proposed a technique of realizing such functions.
1. A mechanism of maintaining a voltage output for a fixed time at the time of interruption of power
2. A mechanism of immediately stopping a voltage output at the time of interruption of power

In view of the above-explained problems, it is an object of the present invention to provide a medical apparatus including both a mechanism of maintaining a voltage output for a fixed time at the time of interruption of power and a mechanism of immediately stopping a voltage output at the time of interruption of power.

To obtain the above object, according to a first aspect of the present invention, there is provided a medical apparatus comprising:
a power supply section which supplies necessary power to respective sections in the medical apparatus;
a maintaining section which maintains supply of power to a part of processing circuits until processing in the processing circuit is completed, when an operation of instructing interruption of supply of power to the respective sections is performed; and
a stopping section which immediately stops supply of power to a part of circuit elements, when an operation of instructing interruption of supply of power to the respective sections is performed.

According to a second aspect of the present invention, there is provided a medical apparatus according to the first aspect, wherein the part of the processing circuits is an image processing circuit and the part of the circuit elements is an imaging element or a display element.

According to a third aspect of the present invention, there is provided a medical apparatus comprising:
a power supply section which supplies necessary power to respective sections;
a main switch which is operable by a user;
an auxiliary switch which is provided in the power supply section and operates in cooperation with the main switch;
a delay circuit provided between the main switch and the auxiliary switch; and
a control circuit provided on a rear stage of the main switch,
wherein, when the main switch is turned off, the control circuit immediately stops supply of power to a part of circuit elements, the delay circuit delays a signal indicative of the turning-off operation for a predetermined time, and the auxiliary switch interrupts supply of power to a part of processing circuits based on the delayed signal.

According to a fourth aspect of the present invention, there is provided a medical apparatus according to the third aspect, wherein the delay circuit includes a CR circuit formed of a resistor and a capacitor, and the delay time is determined based on a time constant of the CR circuit.

### Brief Description of Drawings

FIG. 1 is an external view of an electronic endoscope apparatus to which the present invention is applied;
FIG. 2 is an enlarged view of a processor depicted in FIG. 1;
FIG. 3 is a schematic block diagram of a patient circuit explained above;
FIG. 4 is a schematic block diagram of a processor 4;
FIG. 5 is a view showing a relationship between a main switch, an auxiliary switch, and an output voltage;
FIG. 6 is a view showing a specific structure of a delay circuit 15;
FIG. 7 is a view showing a detailed structure of a power supply device 100;
FIG. 8 is a view for explaining other controls using a signal (OFF, or OFF_P) that is used to judge that the main switch is turned off; and
FIG. 9 is a view showing a structure of avoiding an influence of high-frequency noise.

### Best Mode for Carrying Out the Invention

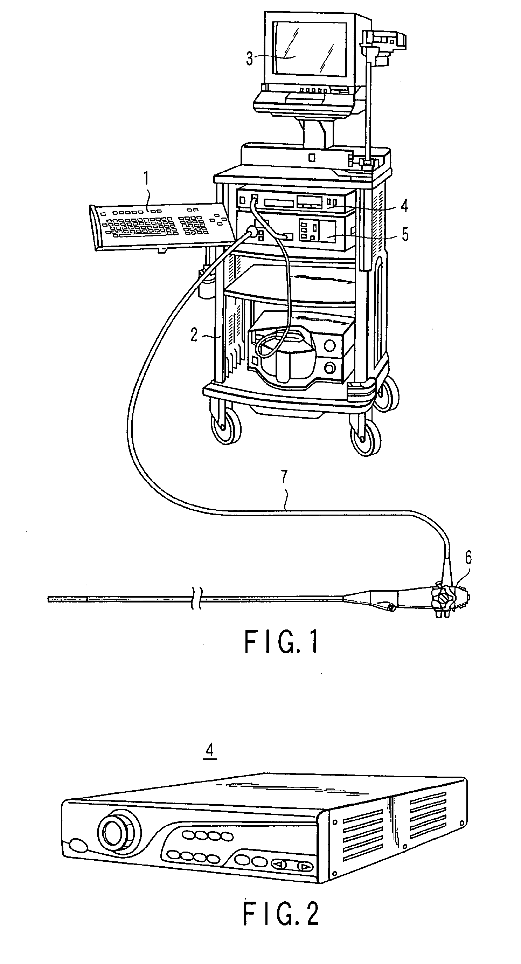

An embodiment according to the present invention will now be explained in detail with reference to the accompanying drawings. FIG. 1 is an external view of an electronic endoscope apparatus to which the present invention is applied, and this apparatus includes a scope 6 which has an imaging element (a CCD) and is inserted into a body cavity of a patient for observation, a light source device 5 which irradiates an affected part, a processor 4 which processes a captured image signal, a monitor 3 which displays the processed image signal, and a keyboard 1 through which data or an instruction is input. The processor 4, the monitor 3, the keyboard 1, and the light source device 5 are provided in an installation rack 2. The scope 6 is connected with the processor 4 through a cable 7. FIG. 2 is an enlarged view of the processor depicted in FIG. 1.

In the above-explained configuration, an image signal captured by the imaging element arranged at a distal end of the scope 6 in a state where the scope 6 is inserted in a body cavity of a patient is input to the processor 4. After signal processing is executed by the processor 4, the image signal is displayed on the monitor 3 or recorded in a non-illustrated recording medium.

Here, since the scope 6 is inserted into the body cavity of the patient for observation, the imaging element in the scope, the cable 7, and a circuit in the processor 4 connected with the cable 7 can be regarded as electrical circuits which may come into contact with the patient. Thus, such an electrical circuit is called a "patient circuit" in terms of electrical safety of the medical device. The patient circuit is electrically insulated to maintain a fixed withstand voltage performance or higher performance from a primary circuit such as commercial power supply, as well as a secondary circuit which is comprised of a grounded sheathed metal part and a signal input/output circuit. This is specified in IEC60601-1 as an international standard of a safety standard for medical devices.

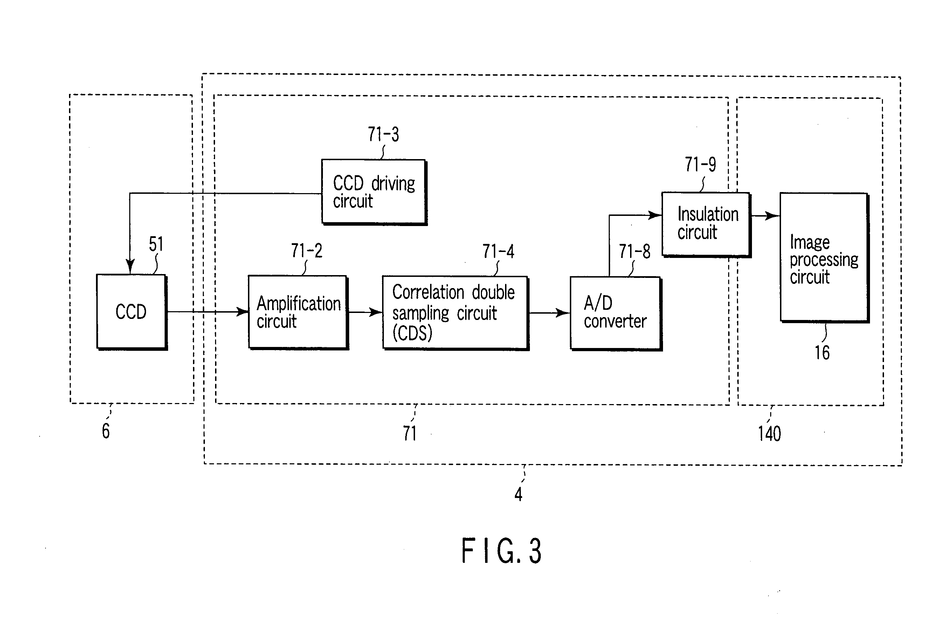

FIG. 3 is a schematic block diagram of the patient circuit 71, and it includes an amplification circuit 71-2 that amplifies an image signal acquired by a CCD 51, a CCD driving circuit 71-3 that drives the CCD 51, a correlation double sampling circuit (a CDS) 71-4 that removes noise from the image signal amplified by the amplification circuit 71-2 and extracts pixel information, an A/D converter 71-8 that converts the image signal from the CDS 71-4 from an analog signal into a digital signal, and an insulating circuit 71-9 that achieves electrical insulation between a patient circuit 71 and a secondary circuit 140 as a grounded sheathed metal and a signal input/output section.

In general, the patient circuit 71, as well as the scope having the built-in CCD 51 as a part of the electrical circuit, can be considered as an electrical circuit which may come into contact with a patient during an operative treatment. Therefore, in terms of electrical safety of the medical device, the patient circuit 71 is electrically insulated at the time of arrangement from the primary circuit such as an AC commercial power supply, as well as from a secondary circuit such as the grounded sheathed metal and a signal input/output section, while keeping a fixed withstand voltage performance or higher performance between them.

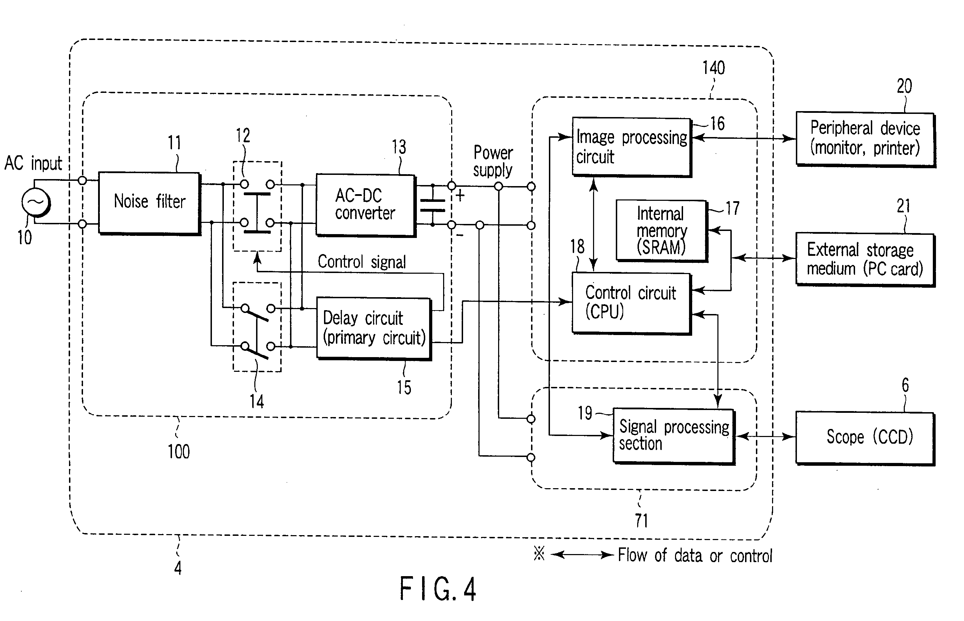

FIG. 4 is a schematic block diagram of the processor 4, and shows signal transmission paths for the power supply unit, the secondary circuit, the patient circuit, and the external connection device such as a scope. FIG. 4 shows a structure which can solve the above-described problem, and is characterized by the inner structure of the processor 4, especially a structure of a power supply device 100.

Such a power supply device 100 includes a noise filter 11, an auxiliary switch 12, a main switch 14 as a power supply switch, an AC-DC converter 13, and a delay circuit 15. A secondary circuit 140 includes an image processing circuit 16, an internal memory (an SRAM) 17, and a control circuit (a CPU) 18.

In the above-explained structure, the AC-DC converter 13 in the power supply device 100 generates a direct-current voltage based on an AC commercial power supply 10, and supplies the direct-current voltage to the secondary circuit 140 and the patient circuit 71 provided on rear stages.

The control circuit (the CPU) 18 in the secondary circuit 140 to which power is supplied processes (e.g., samples) an image signal from the scope 6 based on a command (a control signal) for each operation, or executes I/O communication control with respect to an external connection device.

The image signal input from the scope 6 is converted into a digital signal from an analog signal in a signal processing section 19 in the patient circuit 71, and then transmitted to the secondary circuit 140. Subsequently, this signal is processed into image information by the image processing circuit 16 in the secondary circuit 140, and transmitted to a peripheral device 20, e.g., a monitor or a printer. Likewise, the image information is stored in the internal memory for backup, and stored in a PC card 21 as an external storage medium as required.

An OS (an operating system) is mounted in the control circuit 18 to control a series of operations, e.g., inputting data from the CCD in the scope 6, processing in the patient circuit 71 and the secondary circuit 140, or output to the external storage medium 21 or the peripheral device 20. As types of data processed in this operation, there are image data from the scope 6, intrinsic data concerning a type of the scope 6, patient information data, a communication signal, and a circuit control signal.

Here, if supply of power from the power supply device 100 is stopped for some reason during saving data in the backup internal memory 17 or recording data in the external storage medium 21, i.e., if the main switch 14 of the power supply device 100 is accidentally or intentionally turned off, each processing circuit section cannot normally operate, and the data is destroyed without being stored.

Likewise, if the OS installed in the control circuit 18 is forcibly terminated for some reason, an ending operation is not normally executed, and the data is destroyed.

Thus, according to the present invention, the power supply device 100 mounted in the processor 4 as the image processing device in particular is characterized in that an operation of the power supply device 100 itself is maintained in a fixed time period from a time point at which an OFF operation of the main switch 14 is produced by a user, thereby completing a data saving operation in this time period. The operation of the power supply device 100 is automatically stopped after the fixed time period elapses.

Moreover, the main switch 14 generates two types of judgment signals (OFF_P, OFF) at a timing of OFF so that supply of a power supply voltage that is supplied to the CCD 51 via the patient circuit 71 or a power supply voltage that is used to turn on an LED on a front panel can be stopped simultaneously with turning off the main switch 14. These signals will be explained later.

The details of the power supply device 100 will now be explained with reference to FIG. 4. The power supply device 100 mounted in the medical electronic endoscope apparatus, especially the processor 4 as the image processing device includes the main switch 14 which can be operated by a user, the auxiliary switch 12 which mechanically, electrically, or magnetically operates in cooperation with/simultaneously with the main switch 14, and the delay circuit 15 which instantaneously generates a main switch off operation signal to the CPU 18 as the control signal at a time point where an operation of turning off the main switch 14 is produced and introduces a delay of a predetermined time period from the time point where the operation of turning off the main switch 14 is produced to transmit a signal indicative of turning off the main switch 14 to the auxiliary switch 12.

In the above-explained structure, when the main switch 14 is turned on from a non-active state of the power supply device 100, the delay circuit 15 is activated, and the auxiliary switch 12 and the AC-DC converter 13 operate, thereby starting supply of power to the respective circuit sections provided on the rear stages.

Then, when the main switch 14 is turned off from an active state of the power supply device 100, the delay circuit 15 detects that the main switch 14 is turned off and instantaneously transmits a switch-off signal (OFF, OFF_P) to the control circuit (the CPU) 18 on the rear stage or the CCD driving circuit 71-3 provided in the patient circuit 71, thus starting saving, e.g., image data or an ending operation of the OS. Here, OFF is a secondary circuit judgment signal, and OFF_P is a patient circuit judgment signal. It is needless to say that OFF_P output to the CCD driving circuit 71-3 is a signal that is electrically insulated from the secondary circuit 140 like the patient circuit 71.

Further, the switch-off signal is delayed for a predetermined time and supplied to the auxiliary switch 12 from the delay circuit 15, the auxiliary switch 12 enters an OFF state, and an operation of the AC-DC converter 13 which no longer receives power from the commercial power supply 10 is terminated, thus stopping an output voltage (supply of power to the circuits on the rear stage).

Therefore, the AC-DC converter 13 maintains operation in a period from start of saving/ending in the control circuit 18 to the OFF operation of the auxiliary switch 12, and an output voltage of the power supply device 100 is maintained.

Here, a timing at which the output voltage (supply of power) occurs only when a time determined by the delay circuit 15 in the power supply device 100 (an output maintaining period: T) elapses, and a saving/ending state of the control circuit 18 provided on the rear stage is not monitored. That is, the time of the delay circuit 15 is set in such a manner that the saving/ending operation can be completed within the time determined by the delay circuit 15.

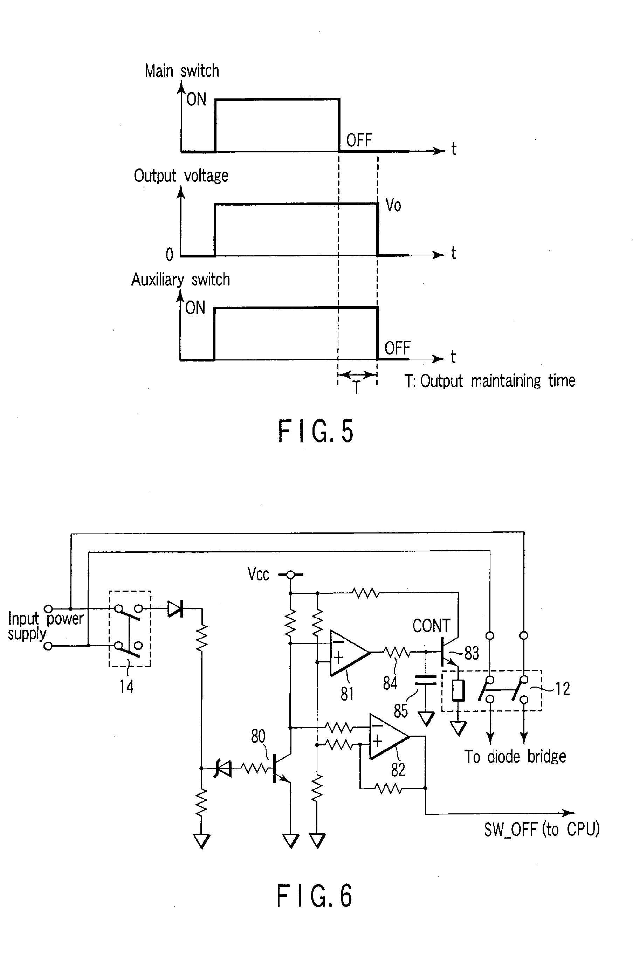

A relationship between the main switch, the auxiliary switch, and the output voltage is as shown in FIG. 5.

FIG. 6 shows a specific configuration of the delay circuit 15. In FIG. 6, whether the main switch 14 is in the ON state or the OFF state is detected by using an input voltage and judged in operational amplifiers 81 and 82 by turning on/off a transistor 80, and supply of driving power to a relay, which is the auxiliary switch 12, is switched by a transistor 83.

In FIG. 6, a circuit which produces a delay time determines an OFF/delay time of the auxiliary switch 12 by using a time constant of a CR circuit formed of a resistor 84 and a capacitor 85. Furthermore, when the main switch 14 is turned off, the OFF signal is immediately transmitted by the operational amplifier 82 to issue a command of starting saving data in the CPU 18 or a command of terminating the OS.

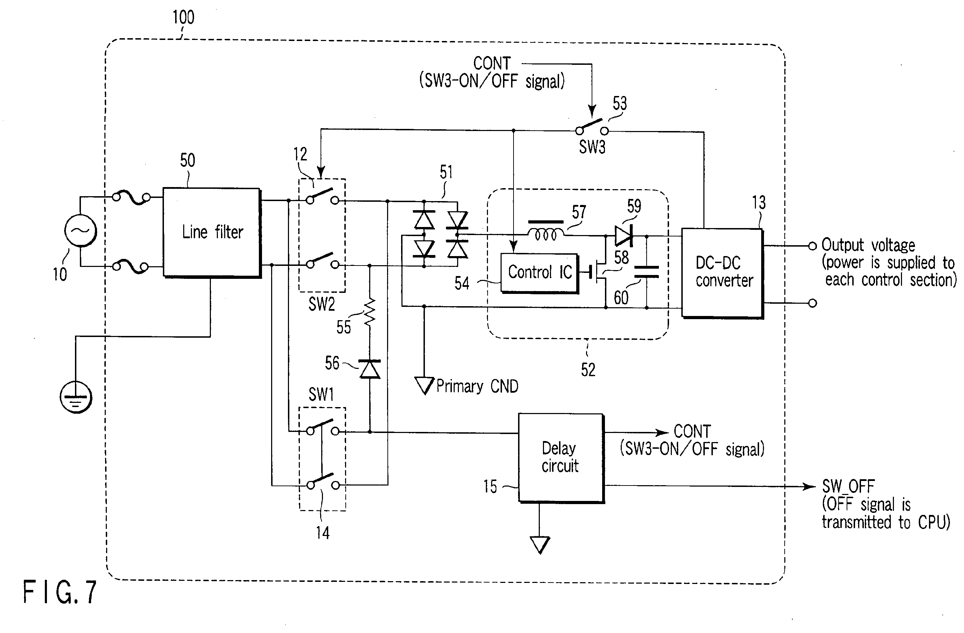

FIG. 7 is a view showing a detailed structure of the power supply device 100. The AC commercial power supply 10 is connected with the main switch 14 which can be turned on/off by a user and the auxiliary switch 12 connected with the main switch 14 in parallel via a fuse and a line filter 50. A resistor R1 (55) which suppresses an incoming current when turning on the main switch and a backflow prevention diode 56 are connected between the main switch 14 and a diode bridge 51 which performs full-wave rectification of an AC voltage waveform. Furthermore, the delay circuit 15 is connected through the main switch 14 to allow supply of a starting voltage of a non-illustrated control IC.

A power factor correction circuit (a PFC circuit) 52 as an active converter that readily realizes power factor correction for a higher harmonic wave suppressing countermeasure is provided between the diode bridge 51 and the DC-DC converter 13. In the drawing, the PFC circuit 52 is formed of a control IC 54, a transistor 58, a diode 59, and a capacitor 60. A merit obtained when providing the PFC circuit 52 lies in that the voltage of the commercial power supply 10 can be either 100V or 200V, voltages used in many parts of the world.

A direct-current voltage converted from an alternating-current voltage by the PFC circuit 52 is converted into a desired voltage by the DC-DC converter 13 and then supplied to the respective circuit sections.

The DC-DC converter 13 has a switching transformer having a primary coil and a secondary coil to achieve electrical insulation. As a switching mode, a flyback mode, a forward mode, and others are generally configured. Here, a soft switching mode which reduces a switching loss and attains a high efficiency is configured in particular.

It is to be noted that a driving power source Vcc in FIG. 6 is the same as that in FIG. 7. Moreover, the transistor 83 as a relay driving power changeover switch depicted in FIG. 6 corresponds to the switch 53 shown in FIG. 7.

In the above-explained configuration, first, when the main switch 14 is turned on by a user, the delay circuit 15 and a non-illustrated PFC circuit section control IC are activated by an input voltage from the commercial power supply 10, an AC voltage fed through the diode bridge 51 is converted into a direct-current voltage by the PFC circuit 52 and supplied to the DC-DC converter 13, then the DC-DC converter 13 is activated to output a desired output voltage.

Here, when the delay circuit 15 is activated, a SW 3 (53) in FIG. 7 is in the ON state, and auxiliary power Vcc from the DC-DC converter 13 is supplied to the relay as the auxiliary switch 12, and the relay enters the ON state.

When the relay as the auxiliary switch 12 enters the ON state, the AC power is supplied to the diode bridge 51 and the subsequent sections via a path including a low-impedance relay with respect to a path of the main switch 14 in which the incoming current suppressing resistor 55 is connected in series, thus continuing the power supply operation state.

Subsequently, when the main switch 14 is turned off by the user from the power supply operation state, the transistor 80 in FIG. 6 enters the OFF state, and an inverting input terminal voltage of the operational amplifier 81 becomes higher than a non-inverting input terminal voltage, an output signal from the operational amplifier 81 has a minus (negative) value, the electric charge stored in the capacitor 85 is discharged in accordance with a time constant with respect to the resistor 84, and the transistor 83 is turned off after a fixed time.

Additionally, when the main switch 14 is turned off, the operational amplifier 82 immediately transmits the switch-off signal to the CPU 18, and data saving in the CPU 18/terminating the OS is executed while the transistor 83 is switched to the OFF state.

When the transistor 83 is turned off, the SW 3 (53) in FIG. 7 is turned off, supply of the driving power to the relay as the auxiliary switch 12 is interrupted, and supply of power from the commercial power supply 10 is stopped. Further, since the control IC 54 of the PFC circuit 52 is also stopped, the DC-DC converter 13 is also stopped, and the output voltage from the power supply device 100 is thereby stopped, thus stopping supply of power to the respective circuit sections on the rear stages.

It is to be noted that the delay circuit may be placed on the secondary side via an isolation section rather than the primary side of the switching transformer as a modification of the foregoing embodiment. Furthermore, a DC power supply may substitute for the commercial power supply as an input.

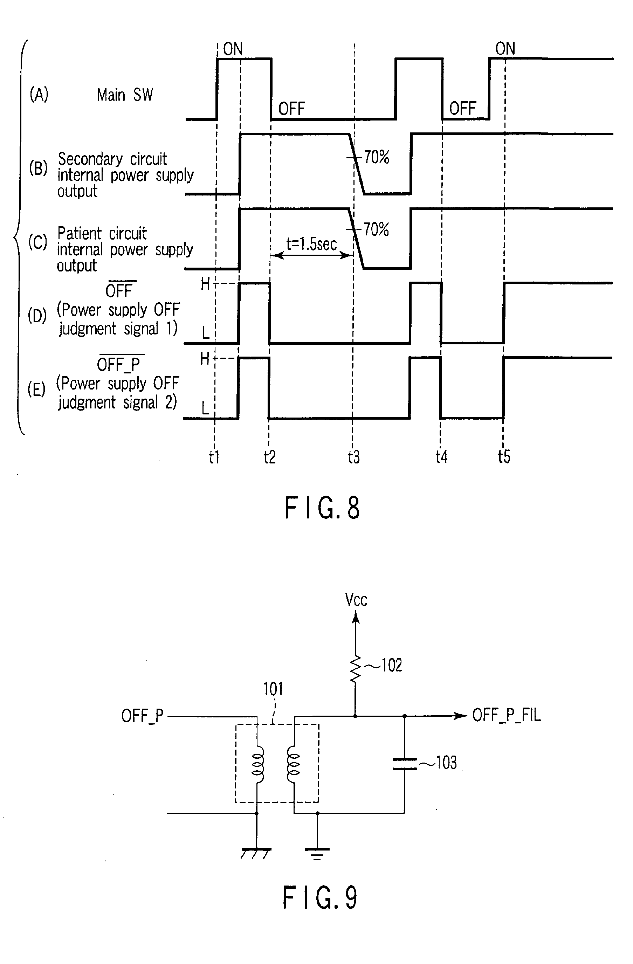

Other controls using the two types of judgment signals (OFF, OFF_P) which are used to judge that the main switch 14 is turned off will now be explained with reference to FIG. 8.

First, since the patient circuit 71 and the secondary circuit 140 are electrically insulated from each other, the OFF signal and the OFF_P signal must be individually output. In the patient circuit 71, upon receiving the OFF_P signal, a power supply voltage which is fed to the CCD can be immediately stopped simultaneously with the OFF operation for the main switch 14 under control of, e.g., a current limiting circuit. That is, in FIG. 8, the main switch 14 is turned on at a time t1 and then turned off at a time t2, and the patient circuit judgment signal (OFF_P) changes to an L level simultaneously with the OFF operation.

Moreover, in the secondary circuit 140, the LED on the front panel is immediately turned off based on the OFF signal. That is, in FIG. 8, the main switch 14 is turned on at the time t1 and then turned off at the time t2, and the secondary circuit judgment signal (OFF) changes to the L level simultaneously with the OFF operation. As a result, a user does not have an unnatural sense at the time of turning off the power supply.

Additionally, a secondary circuit internal power supply output and a patient circuit internal power supply output do not change to the L level simultaneously with the OFF operation for the main switch 14, but they are reduced to an approximately 70% level with a predetermined delay time t = 1.5 second (a time t3) and then change to the L level. Based on execution of such control of delaying interruption of the internal power supply voltage, the image processing circuit can normally complete the recording operation even if the main switch 14 is turned off during execution of electronic recording of an image in, e.g., a PC card, and shutdown processing can be subsequently performed, thereby avoiding loss of an inspection image. Further, executing such delay control of the power supply shutdown can prevent the scope from being removed while receiving supply of power, thus improving safety.

Furthermore, although the OFF_P signal is the signal that is used to judge the OFF state of the main switch 14 with respect to the patient circuit 71 and a supply voltage or a driving pulse for the CCD is carried out based on this judgment signal, the OFF_P signal is a signal that is electrically insulated with respect to the earth, and hence it is easily affected by high-frequency noise of, e.g., a cautery knife. Therefore, stabilization based on such a structure as shown in FIG. 9 is achieved in order to avoid occurrence of a problem, e.g., loss of an image during use of a cautery knife. The structure shown in FIG. 9 is formed of a transformer 101, a resistor 102, and a capacitor 103.

### Industrial Applicability

According to the present invention, even if power supply is accidentally interrupted by a user, data is assuredly saved. Moreover, safety can be assured, and a sense of use is not impaired for a user.

## Claims

1. A medical apparatus comprising:
a power supply section which supplies necessary power to respective sections in the medical apparatus;
a maintaining section which maintains supply of power to a part of processing circuits until processing in the processing circuit is completed, when an operation of instructing interruption of supply of power to the respective sections is performed; and
a stopping section which immediately stops supply of power to a part of circuit elements, when an operation of instructing interruption of supply of power to the respective sections is performed.

2. The medical apparatus according to claim 1, wherein the part of the processing circuits is an image processing circuit and the part of the circuit elements is an imaging element or a display element.

3. A medical apparatus comprising:
a power supply section which supplies necessary power to respective sections;
a main switch which is operable by a user;
an auxiliary switch which is provided in the power supply section and operates in cooperation with the main switch;
a delay circuit provided between the main switch and the auxiliary switch; and
a control circuit provided on a rear stage of the main switch,
wherein, when the main switch is turned off, the control circuit immediately stops supply of power to a part of circuit elements, the delay circuit delays a signal indicative of the turning-off operation for a predetermined time, and the auxiliary switch interrupts supply of power to a part of processing circuits based on the delayed signal.

4. The medical apparatus according to claim 3,
wherein the delay circuit includes a CR circuit formed of a resistor and a capacitor, and the delay time is determined based on a time constant of the CR circuit.
